# EUROPEAN PATENT SPECIFICATION

(11) **EP 4 062 170 B1**
(45) Date of publication and mention of the grant of the patent: **16.10.2024**
(21) Application number: 20810939.7
(22) Date of filing: 19.11.2020
(51) Int. Cl.: G01N 30/82, B01D 15/24, B01D 15/38, C07K 1/22

(54) **METHOD FOR CONTROLLING PURIFICATION SYSTEM**
VERFAHREN ZUM STEUERN EINES REINIGUNGSSYSTEMS
PROCÉDÉ DE CONTRÔLE D'UN SYSTÈME DE PURIFICATION

(30) Priority: 21.11.2019 GB 201916961
(43) Date of publication of application: 28.09.2022
(73) Proprietor: Cytiva BioProcess R&D AB, 751 84 Uppsala (SE)
(72) Inventor: SÖRBY, Lennart, 751 84 Uppsala (SE)
(74) Representative: Démoulin, Eva Lotta
(86) International application number: PCT/EP2020/082687
(87) International publication number: WO 2021/099465

(56) References cited:
- WO-A1-2019/016154
- US-A1- 2004 216 510

## Description

### TECHNICAL FIELD

The present invention relates to a method for controlling fraction collection of a target product in a chromatography system having one or more chromatography columns.

### BACKGROUND

Liquid chromatography is one of the most commonly used separation principles in the analysis and manufacture of biomolecules, such as proteins and peptides. There are numerous commercial instruments and different chromatography formats available for the processing of biomolecules by chromatography.

Currently, the clarified or clear feed from the bioreactor is introduced into a column capture chromatography system configured for a cyclic purifying process to extract the target product. The cyclic process includes: loading the feed onto a column, washing the column, eluting the target product and thereafter cleaning the column before the column is loaded with new feed. For purification processes using small volume columns the elution cycle will be rather short and the window for capturing the target product during elution have to be detected by the UV sensor at the outlet of the columns using a fast update frequency. However, the drawback of UV sensors is the limited resolution and thus the capture of the target product during elution may be inefficient.

US-2004/216510 discloses a method and apparatus for controlling fraction collection in an eluent stream flowing from a liquid chromatography column, wherein a first triggering detector recognizes the presence of a target substance in the eluent flow and initiates a delay time to trigger the fraction collector.

WO-019/016154 discloses a process and system for the cyclic purification of a product biomolecule using a chromatography step, wherein the collection of flow-through is started at a predetermined first concentration of the product biomolecule in the flow-through and is stopped at a predetermined second concentration.

Thus, there is a need to improve the process to detect the presence of target product during elution, especially for small volume columns in purification using a column capture chromatography system.

### SUMMARY

An object of the present disclosure is to provide methods and devices configured to execute methods and computer programs which seek to mitigate, alleviate, or eliminate one or more of the above-identified deficiencies in the art and disadvantages singly or in any combination.

The object is achieved by a method, a system, a computer program and a computer-readable storage medium as defined by the independent claims.

An advantage is that the an UV detector with high resolution, i.e. fast update frequency, is not needed.

Another advantage is that the yield and quality of the captured target product is improved.

Further objects and advantages may be obtained from the detailed description by a skilled person in the art.

### BRIEF DESCRIPTION OF THE DRAWINGS

Fig. 1 illustrates an overview of a bioprocess purification system designed to purify a target product from a harvest fluid from a bioreactor.
Fig. 2 illustrates the concept of controlling upstream/downstream processes in a bioprocess purification system.
Fig. 3a illustrates an ideal cyclic capture chromatography process configured to deliver at least one product from a volume of sample feed using a purification unit with a cyclical repetitive purification process.
Fig. 3b illustrates a schematic representation of a chromatography system.
Fig 4. is a schematic illustration of one possible cyclic mode of operation of a chromatography system
Fig 5a and 5b show graphs illustrating the basic concept of the improved process to capture the target product in a chromatography process
Fig. 6 illustrates a method for controlling faction collection of a target product in a chromatography system.

### DETAILED DESCRIPTION

A bioprocess purification system is designed for production and purification of target products (such as proteins, biomolecules from cell culture/fermentation, natural extracts) by growing cells capable of expressing the target product in a cell culture bioreactor followed by a downstream purification process (also referred to as Downstream process) for purifying the target product. The downstream purification process may be any suitable process capable of providing a purified target product, the process may comprise one or multiple steps. One commonly used step in a downstream purification process is chromatography. In particular the current invention relates to a bioprocess purification system arranged to produce and provide a purified product during an extended period of time from a volume of sample feed. The product is provided as a batch with a volume larger than the volume of the column, or the product is harvested from the bioreactor and purified by the downstream purification process while the cell culture is maintained. This type of cell culture is herein referred to as "continuous cell culture process" and examples of such cell cultures includes perfusion cell culture and chemostat cell culture.

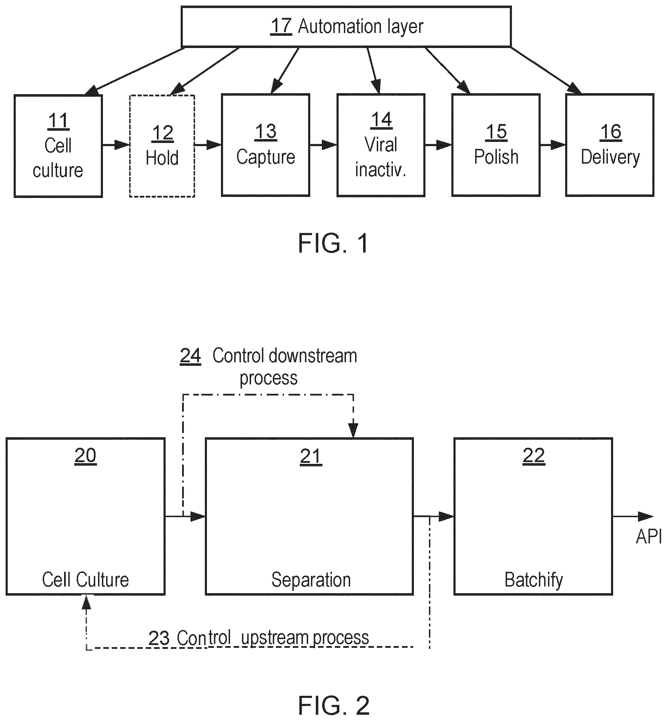

In figure 1, an overview of a bioprocess purification system according to one embodiment, configured to purify a target product using a separation process is shown. The bioprocess purification system comprises a number of process steps related to Cell culture 11, Hold 12, Capture 13, Viral inactivation 14, Polish 15 and Delivery 16.

In one of the disclosed embodiments of the present invention the cell culture step 11 may be a continuous cell culture process which comprises of continuous addition of nutrients and continuous removal of product and waste over an extended period of time (harvest). The process can either be operated in perfusion retaining the cells in the bioreactor by e.g. using an Alternate Tangential Filtration (ATF) device. Alternatively, the bioreactor is operated without cell retention, i.e. a chemostat. The cell culture step may comprise process control for viable cell density, VCD, but also nutrients and metabolites. The VDC, productivity and product quality may be controlled by adapting the components of the cell culture media fed to the culture or by addition of certain components directly to the culture, as described in more detail below.

In some embodiments, the harvest containing the target product may be clarified before feeding the harvest to the downstream purification process, e.g. by filtration, centrifugation or another technique.

The hold step 12 is an optional step depending on process needs, e.g. if a filter is in-line before capture step 13. The step may comprise process control on weight, and the next step in the process starts when a pre-determined volume value is reached, or alternatively after a certain time period or when a pre-determined mass is reached. The hold step may be used for collecting a volume of filtered feed from a perfusion cell culture.

In another embodiment of the present invention, the cell culture step is omitted and a batch of sample feed containing the product is provided in the hold step 12 and provided to the purification process.

In the disclosed embodiment, the downstream purification process comprises three steps Capture 13, Viral inactivation 14 and Polish 15. The capture step 13 comprises a chromatography process either in a single chromatography column or a plurality of chromatography columns connected in parallell or series or operated sequentially. A filter may be provided in-line before the capture step. The capture step comprises multiple batch elutions, and process control e.g. using in-line UV-sensors handles variation in feed concentration and resin capacity. The next step starts when a pre-determined amount value (e.g. volume, mass or time) is reached.

In the viral inactivation step 14, different options for virus inactivation is available depending on process needs. One option is to use batch mode with low pH for 30-60 minutes in a hold up tank. The step may comprise process control on volume, time, temperature and pH. The next step starts when a pre-determined time is reached.

The polish step 15 may be straight through processing (STP) with a connected batch step or continuous chromatography with a continuous load step, or a combination thereof. The flow rate is adjusted to perfusion rate required by producer cells, which means that the flow rate is determined by the preceding step. The step may comprise process control for UV, flow and volume, and the next step starts when a pre-determined volume and amount is reached, alternatively when a timeout is reached.

The delivery step 16 may comprise a virus removal step, e.g. a viral filter, before an ultrafiltration step. The delivery step may be used as concentration step for batch addition of processed harvest from polish step. The delivery step 16 may comprise continuous or batch delivery of product and may comprise continuous or batch removal of waste. The step may comprise process control for pH, conductivity, absorbance, volume and pressure, and delivery is achieved when a pre-determined product concentration in a pre-defined environment is reached.

An automation layer 17 is used for handling decision points for next step in the process. Different type of sensors (not shown), both in-line sensors and off-line sensors, are integrated into the process flow to monitor different parameters that may be used for providing the automation layer 17 with data that could be used to handle the decision points. Sensors include but are not limited to only measure flow, VCD, weight, pressure, UV, volume, pH, conductivity, absorbance, etc.

It should be noted that UV absorbtion is an example of a parameter that could be monitored to detect the composition of the harvest being purified. However, other parameters may be used operating in other frequency ranges, such as IR, fluorescence, x-rays, etc.

The product quality of the target product produced in a bioprocess purification system may be improved by obtaining information related to the target product during the process run, or the produced target product itself. Attributes relevant to product quality have to be measured, and different analytic methods may be used such as Mass Spectroscopy, MS, Light Scattering, Size Exclusion Chrom, SEC, Raman spectroscopy, etc.

The cell culture system comprises a bioreactor that produces a harvest containing the target product and the cell culture process may be controlled to optimize the product quality of the target product. Examples of parameters that may be controlled in the bioreactor is temperature, aeration, agitation etc.

Figure 2 illustrates the concept of controlling upstream/downstream processes in a bioprocess purification system. The illustration of the bioprocess purification system is simplified and comprises three steps: Cell culture 20, Separation 21 and batchify 22. The target product (in this example exemplified by "active pharmaceutical ingredient" - API) is delivered after the batchify step.

The Cell culture step 20 may be a continuous cell culture process as described above that includes continuous addition of nutrients to e.g. a cell perfusion process with continuous harvest of target product and waste or a batch of sample feed provided in a volume larger than the capacity of the single column chromatography system. The sample feed, comprising product and waste, is considered to be the harvest that is fed into the Separation step 21 which may include one or more steps of a downstream purification process. The separation step comprises a process for at least partly separating the product from the waste in the harvest and the product is forwarded to the final step Batchify 22, in which the product is handled to be ready for delivery as API.

After the separation step, certain parameters, or quality attributes, may be measured, e.g. composition of impurities in target product or amount of fragments or aggregates of the target product using mass spectrometer, MS, or spectrometry. This information may be used to control an upstream process 23. For instance, if a high amount of degraded target product is detected after separation, this may be counteracted by changing parameters in the cell culture step, e.g. by an increased flow rate of medium into the bioreactor to prevent degradation of target product molecules before introduced into the separation step 21. Alternatively, feeding of nutrients or process parameters in the cell culture may be adjusted based on the measured quality attributes, as described in more detail below. Variations in composition of the sample feed that is provided to the separation step 21, may be detected after the column, e.g. if breakthrough of the captured product is detected, which may be counteracted by changing the amount of sample feed loaded onto the column.

The same concept may be used to control a downstream process 24. The concentration of target product in the harvest being fed into the separation step 21 may be determined by measuring the time to load each column and the peak amount of target product after elution. This information may be used to adjust the elution based on the concentration of target product in the harvest being fed into the separation step.

The chromatography device may be any suitable type of chromatography device, such as a conventional packed bed chromatography column, a nanofiber device, a membrane holder device, membrane chromatography device, monolith or chromatography cassette, radial flow column, etc.

Moreover, the term chromatography device as used herein may also be comprised of two or more chromatography units connected and operated in parallel. In one embodiment, the chromatography device is a nanofiber separation device, where nanofiber sheets are generally attached in a plastic device. In general terms, the chromatography device has an inlet and an outlet for liquid to pass through the device and an affinity matrix in the form of one or more of a resin, membrane, nanofiber sheet, monolith etc. there between. The design of the device is such that the liquid distributes evenly in the affinity matrix and that the device preferably has a low dead volume.

According to one embodiment, the chromatography device is a nanofiber device as disclosed in patent applications US2014/0296464, US2016/0288089 and WO2018/037244. The pore size of the nanofiber material allows high flow rates providing residence time down to a few seconds.

The chromatography device may have a chromatography column bed volume of 0.1 mL to 5 L.

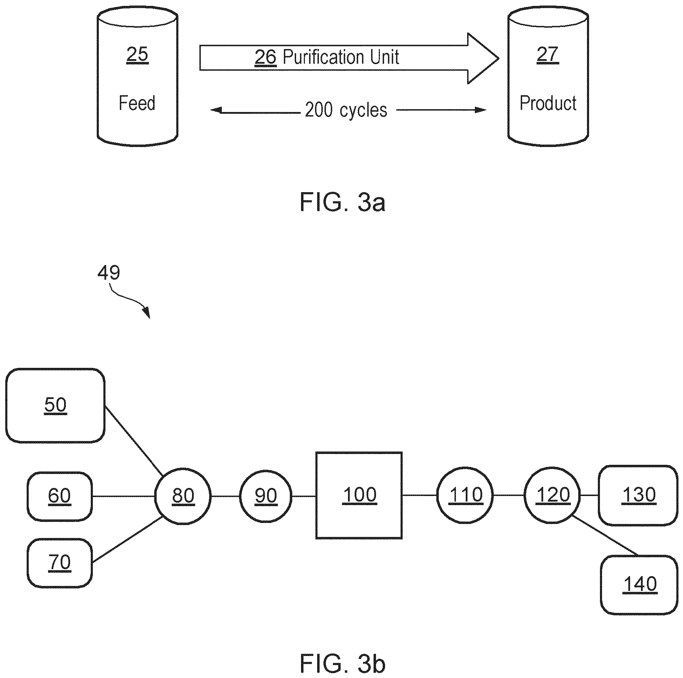

Figure 3a illustrates an ideal cyclic capture chromatography process configured to deliver at least one product 27 from a volume of sample feed 25 using a purification unit 26 with a cyclical repetitive purification process. In this example the purification unit comprises only one column and the same column is used over 200 cycles, wherein each purification cycle comprises loading an amount of sample feed onto the column, washing the column and eluting the at least one captured product. However, in most real life situations the performance of the system deteriorate as a function of cycles performed, and when the performance is decreased, the column may be cleaned using a Cleaning-in-place procedure, CIP, in order to at least partially restore the performance of the chromatography column. Also, when the performance of the column is not restored properly by the CIP, the column may have to be replaced by a new column in order to keep an efficient process running. Methods for controlling the performance of a cyclic capture chromatography process are presented in the co-pending patent applications WO 2020/173862 and GB1909274.1.

Fig. 3b shows a schematic representation of a chromatography system 49 comprising a source of sample solution 50, a source of elution buffer 60, a source of cleaning solution and buffer/wash buffer 70, an inlet valve 80, a pump 90, a chromatography column 100, a detector 110, an outlet valve 120, a product receptacle 130 and a waste receptacle 140.

As discussed above, the source of sample solution 50 may be a continuous cell culture, optionally intermediate clarification etc. or a batch container.

The source of elution buffer 60 and the source of cleaning solution 70 may be containers of suitable size comprising the relevant solutions and depending on the specific purification process one or more (or less) solutions may be provided. Alternatively, one or more of said solution sources 60, 70 may be provided as an in line mixing or dilution unit where one or more of said solutions are prepared and provided on demand.

The inlet valve 80 and the outlet valve 120 may be of any suitable type, typically rotary valve which can achieve thousands of turns, with quick transitions, for multiple flow paths. Other examples include solenoid valve, pinch valve, pneumatic valve etc.

The pump 90 may be reciprocating piston pumps, to provide a consistent flow of the mobile phase. Other pumps could be a peristaltic pump, a centrifugal pump, diaphragm pump, gear pump, screw pump, etc.

The chromatography column 100 is a microporous resin column, nanofiber device, membrane, monolith, etc.

The detector 110 may be one or more detectors that is/are capable of providing a signal indicative of the precense of the target product in the fluid flow, such as a UV detector, a light scattering detector, pressure sensor, diode array detector, fluorescence detector (or any spectroscopic detector...), a pH meter, a conductivity detector, ...etc.

The product receptacle 130 is arranged to collect the product eluted from the column and it may be any suitable container. Alternatively, the chromatography system may be directly (or indirectly through a hold tank) connected to a downstream process as discussed above, whereby the product receptacle 130 is the inlet of said downstream process.

When operating a chromatography system 49 in a cyclic mode, wherein a small volume of product is captured and eluted in multiple cycles, it becomes more important to reduce the overall hold up volume of the chromatography flow path compared to a batch type system where the chromatography column has a large volume and capacity. In cyclic operation the overall process economy is highly dependent on the volume of process solutions consumed and in particular any residual volumes of process solutions that remain in the system after switch to a subsequent process solution due to the hold-up volume of the system and which residual volumes are not directly needed for the chromatography process. Moreover, it is desirable to keep the flow path in between the outlet of the chromatography column 100 and the outlet valve 120 for selectively directing the eluted product to the product receptacle 130 as short as possible in order to avoid broadening of the elution peak due to diffusion, which may lead to reduced product concentration and purity.

In order to achieve high productivity in a chromatography system 49 operating in cyclic mode it is further desirable to operate the system at higher flow rates and back pressure levels compared to a batch type system. This is the case with nanofiber devices where the productivity may be in excess of 100 g/L/hr, when compared with classic batch chromatography 1-20 g/L/hr.

For example a UV detector with a frequency of 64 reads per second, at a productivity of a classical batch chromatography of 6 g/L/hr, a 3 column volume (CV) elution peak will have 4608 measurements. At a flow rate high enough for to achieve 300 g/L/hr, this drops to 92 measurements, as the residents time within the unit becomes 0.48 seconds. This reduction in measurements could lead to delays in detection and product loss.

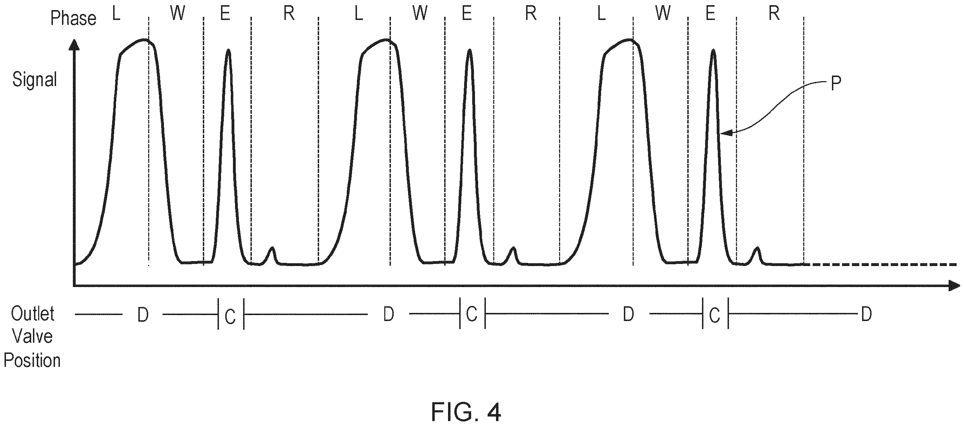

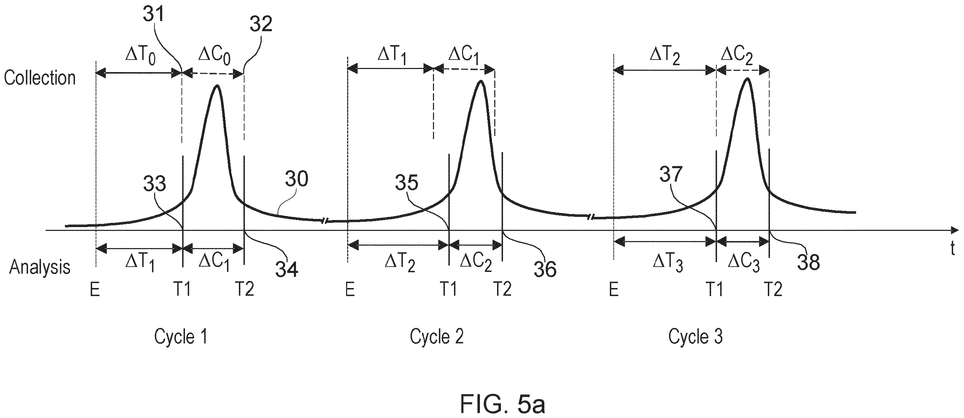

A method for controlling fraction collection of a target product in a chromatography system is disclosed. The chromatography system is provided with at least one affinity chromatography separation unit and is configured for cyclic purification performed on a sample comprising the target product. Cyclic purification is defined as a purification process that is performed in consecutive cycles, as illustrated in Fig 4 and 5a. Each cycle of the purification process comprises: loading feed onto the separation unit, washing the separation unit, and eluting the target product from the separation unit by providing a flow of an elution fluid over the separation unit. A fraction of the outlet flow from the separation unit containing the eluted target product is selectively directed to a subsequent production step, such as product collection vessel or a consecutive production process.

Fig 4 is a schematic illustration of one possible cyclic mode of operation of a chromatography system 49 comprising the phases Load (L), Wash (W), Elute (E) and Regeneration (R). The solid line represents the signal recorded by the detector 110 and where purified product is represented as the sharp peak P. Under the time axis the operation status of the outlet valve 120 is shown, where D indicates that it is arranged in discard (or waste) position and C in product collection position. As is evident from fig. X, the time frame for collecting the elution peak when the system is operated at high flow rate is very limited and taken the steep front and trailing edges of the peak P, the timing of the valve switching becomes critical in order to achieve efficient collection of the product. In case the switch from D to C is too early and from C to D too late, the collected fraction will be diluted and possibly contain more impurities. Whereas in case the switch from D to C is too late and from C to D too early, the collected fraction will more concentrated and contain less impurities, but valuable product will be directed to waste and be discarded. As mentioned, the flow path from the outlet of the chromatography column 100 to the outlet valve 120 is preferably short, whereby the time-frame available for switching the outlet valve 120 in response to the signal level registered by the detector 110 for the peak P may not be sufficient to achieve proper timing of the switching.

The method could be applicable for any system where there are repeated purification cycles and wherein the cycles are fast and the flow rate high such that it may be difficult to read the product concentration using a sensor (UV adsorbtion signal or the like) and in direct response control a valve to discard or collect the flow from the system as purified product. Examples of such systems may be chromatography systems that are arranged to operate in a cyclic mode, and where the fluid flow rate during elution is high and the flow path between the sensor registering the product concentration and the selection device (e.g. a valve) that is arranged to direct the fluid flow for collection or to waste.

Figure 5a illustrates a graph with consecutive elution cycles, cycles 1-3. The curve 30 is the UV adsorbtion signal corresponding to the content of target product in the output flow during elution. The symbol "E" denotes the start of the elution phase for each cycle, ΔT₀ is the time duration before capture of target product in the cycle 1 starts at the start trigger point 31. A fraction of the elution containing the target product is captured during a first time period ΔC₀, i.e. until the end trigger point 32, as illustrated in Fig 3, cycle 1 over the graph denoted "Collection".

Cycle 1 is the first cycle, which is normally only used for calibration and the trigger points 31 and 32 may be set by the operator before the process starts or the start trigger point 31 is set when the UV absorbtion signal increases and reaches a predetermined threshold value and the end trigger point 32 is set when the UV absorbtion signal decreases and reaches a predetermined threshold value (which threshold value can be the same as for the first trigger point).

Thus the first time period, may be broader than normally allowed in order to be sure to capture the target product during elution and calculate new trigger points 33 and 34 to determine ΔT₁ and the next time period ΔC₁ for the next cycle. This is illustrated in Fig 5a, cycle 1 under the graph denoted "Analysis". Start trigger point 33 and stop trigger point 34 are identified by evaluating the timing of the captured fraction of the elution based on the amount of target product in the outlet flow during cycle 1 as well as captured impurities.

The timing is applied to the next cycle by waiting ΔT₁ after "E" before starting to collect target product during elution phase in cycle 2. A fraction of the elution containing the target product is collected during the time period ΔC₁. New trigger points 35 and 36 are thereafter calculated, as illustrated in Fig 5a, for cycle 2 to determine ΔT₂ and the next time period ΔCz for the next cycle. Start trigger point 35 and stop trigger point 36 are identified by evaluating the timing of the captured fraction of the elution based on the amount of target product in the outlet flow during cycle 2 as well as captured impurities.

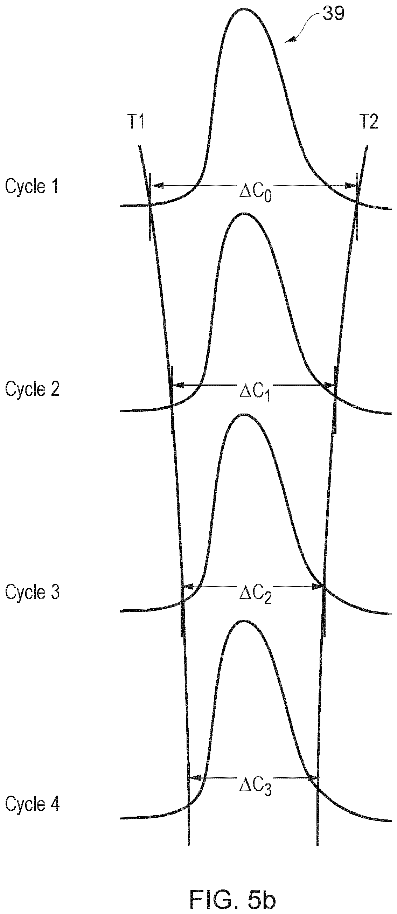

The timing is applied to the next cycle by waiting ΔT₂ after "E" before starting to collect target product during elution phase in cycle 3. A fraction of the elution containing the target product during collected is the time period ΔCz. New trigger points 37 and 38 are thereafter calculated, as illustrated in Fig 5, cycle 3 to determine ΔT₃ and the next time period ΔC₃ for the next cycle. Start trigger point 37 and stop trigger point 38 are identified by evaluating the timing of the captured fraction of the elution based on the amount of target product in the outlet flow during cycle 3 as well as captured impurities.

The calculated start trigger points 33, 35 and 37 are commonly denoted T1, and the calculated stop trigger points 34, 36 and 38 are commonly denoted T2.

Figure 5b is an illustration of the changed timing for consecutive cycles, wherein the timing of the consecutive cycle is calculated based on the yield and amount of impurities captured in the present cycle. As illustrated, during the first cycle "Cycle 1" elution is captured during an initial time period ΔC₀, wherein the captured fraction of the elution may be used for calibration purposes to determine the timing of the elution during the elution phase for the following cycle. In this example the trigger points T1 and T2 are selected to ensure that the elution peak 39 is within the time period ΔC₀ and 100% of the target product is captured together with impurities.

In order to reduce the amount of impurities, new trigger points are applied and a time period ΔC₁ for the next cycle "Cycle 2" is calculated and a fraction of the elution containing the target product is collected during that time period. This process is repeated for "Cycle 3" and during "Cycle 4", the amount of target product in the fraction of the collected elution during time period ΔC₃ may be reduced, e.g. to 95% of the amount of target product, while the amount of impurities is lower compared to the previous cycles.

An important part of the invention is to identify the starting point for collection of target product. However, there are some options for determining the optimal stop point. According to some embodiments the stop point is determined based on the duration from the starting point, which is adapted over time by peak analysis. The duration may be actively controlled by determining the duration from the starting point to the peak intensity and then estimating the stop point based on that, i.e. there will be a level of pre-determination of the stop point which may be sufficient in order to have a more direct control methodology.

Thus, the method for controlling fraction collection of the target product comprises determining trigger points for target product collection in relation to presence of target product in the outlet flow, e.g. using an UV detector, during the initiation of the elution phase which are cyclically repeated.

It should be noted that the process described in connection with Figures 5a and 5b may be used for any type chromatography system, i.e. not only single column systems, where there are repeated purification cycles and wherein the cycles are fast and the flow rate high. In such systems it may be difficult to read the product concentration using a sensor (UV adsorbtion signal or the like) and in direct response thereto control a valve to discard or collect the flow from the system as purified product.

Table 1 below illustrates residence time for separation units with different residence time and the corresponding resolution of the UV detector (i.e. reads per elution peak). As illustrated, it will be difficult to monitor the elution peak and adjust the timing of the capture phase purely based on the signal from the UV detector for separation units with low residence time.

**Table 1**

| | Residence time (sec) | CV/sec | elution peak (4 CV) in time (sec) | Reads per second | Reads per elution peak |
|---|---|---|---|---|---|
| 6 min | 360 | 0.003 | 1440 | 10 | 14400 |
| 3 min | 180 | 0.006 | 720 | 10 | 7200 |
| 1 min | 60 | 0.017 | 240 | 10 | 2400 |
| 30 sec | 30 | 0.033 | 120 | 10 | 1200 |
| 10 sec | 10 | 0.100 | 40 | 10 | 400 |
| 5 sec | 5 | 0.200 | 20 | 10 | 200 |
| 2 sec | 2 | 0.500 | 8 | 10 | 80 |
| 1 sec | 1 | 1.000 | 4 | 10 | 40 |

CV stands for Column Volume, it is the internal volume of the separation unit. In this example, the elution peak is set to 4 CV in width. From a regular bead column (at the recommended residence time of 6 minutes), 14400 data points per elution peak is expected, compared with a separation unit running at a 1 second residence time having 40 data points for the same elution peak.

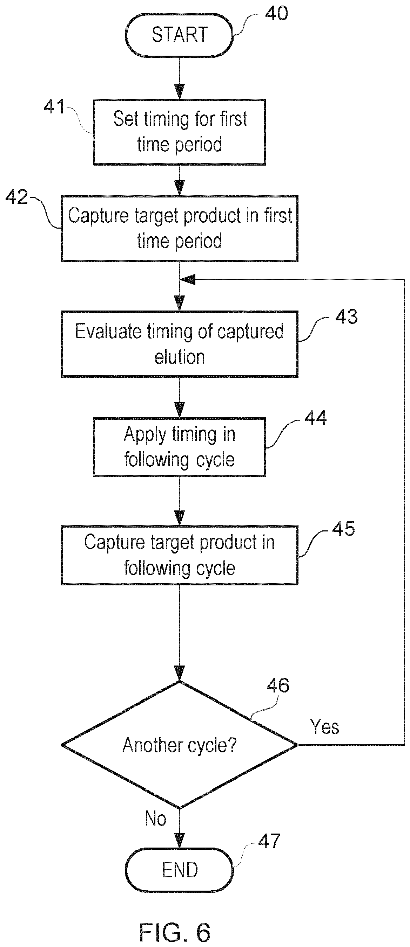

Figure 6 illustrates a method for controlling fraction collection of a target product in a column chromatography system with at least one affinity chromatography separation unit. The chromatography system is configured for cyclic purification performed on a sample comprising the target product, each cycle of the purification process comprises: loading feed onto each separation unit, washing each each separation unit, and eluting the target product from each separation unit by providing a flow of an elution fluid over each separation unit and selectively directing a fraction of the outlet flow from each separation unit containing the eluted target product to a subsequent production step.

The method for controlling fraction collection of target product further comprising:
Setting 41 a first time period, ΔC₀, based on the trigger points, 31 and 32, within which a fraction of the elution in the first cycle is captured 42.

Evaluating 43 timing of the captured elution to identify next time period ΔC₁, ΔC₂, ΔC₃.

Applying 44 the timing to capture a fraction of the elution during the elution phase in the following cycle.

Collecting 45 the captured fraction of the elution in the following cycle for collection. It should be noted that the term "collection" covers both the occasion when the fraction is collected in a collection vessel and when the fraction is directed to a subsequent production step. Ideally, the fraction contains the target product, but in practice since the eluted target product will exit the column as a "gradient peak" there will always be a balance between collecting all target product (yield) versus avoiding collection of other components (impurities), or reducing the eluate concentration.

If more target product is determined to be collected, step 46, the process continue with repeating steps 43-45 during the purification process to capture a fraction of the target product during the elution phase of the following cycle.

If not, the process ends in step 47.

According to some embodiments, elution captured in the first cycle "cycle 1" in step 42 is used for calibration purposes to determine the timing of the elution during the elution phase of the following cycle "cycle 2". According to some embodiments, the elution captured in the first cycle is collected, or discarded as waste to reduce the risk for contamination of impurities during capture.

According to some embodiments, the chromatography system is a single column chromatography system.

According to the invention, the method further comprises registering the presence of the target product in the outlet flow from each column over time, e.g. in an elution curve 30, and the step of determining trigger points further comprises determining start time T1 and a stop time T2 for target product collection.

According to some embodiments, the presence of the target product over time is registered as an elution curve, and the initiation of the elution phase is determined from the elution curve 30.

The invention also relates to a chromatography system comprising at least one affinity chromatography separation unit configured for cyclic purification performed on a sample comprising a target product and a control unit configured to control each cycle of the purification process, wherein the control unit is further configured to perform the method described in connection with figure 6.

The methods described above may be implemented in a computer program for controlling a bioprocess purification system. The computer program comprises instructions which, when executed on at least one processor, cause the at least one processor to carry out the method according to the different variations described in connection with figure 6. The computer program for controlling the bioprocess purification system may be stored on and carried by a computer readable storage medium.

In one embodiment, the switching of the outlet valve 120 is controlled by a combined algorithm where:
If the detector signal is higher than a predetermined level before T1, the valve is opened, and
If the detector signal is lower than a predetermined level before T2, the valve is closed.

## Claims

1. A method for controlling fraction collection of a target product (27) in a column chromatography system (49) including at least one affinity chromatography separation unit, the chromatography system (49) being configured for cyclic purification performed on a sample comprising the target product (27), each cycle of the purification process including:
- loading feed (25) onto the separation unit,
- washing the separation unit, and
- eluting the target product from the separation unit by providing a flow of an elution fluid over the separation unit and controlling fraction collection by selectively directing a fraction of the outlet flow from the separation unit containing the eluted target product to a subsequent production step,
wherein the method for controlling fraction collection of the target product comprises:
registering the presence of the target product (27) in the outlet flow from each column over time,
determining trigger points for target product collection in relation to presence of target product (27) in the outlet flow, the trigger points comprises a start time T1 and a stop time T2 for target product collection in relation to the start timepoint E of the elution phase for each cycle, and wherein the method for controlling fraction collection of target product further comprises:
a) setting (41) a first time period ΔC₀ based on the trigger points (31, 32) within which a fraction of the elution in the first cycle is captured (42);
b) evaluating (43) timing of the captured elution to identify trigger points (33, 34, 35, 36, 37, 38) and the corresponding next time period ΔC₁, ΔC₂, ΔC₃;
c) applying (44) the timing to capture and collect a fraction of the elution during the elution phase in the following cycle;
d) repeating steps b) - c) during the purification process.

2. The method according to claim 1, wherein elution captured in the first cycle is used for calibration purposes to determine the timing of the elution during the elution phase for the following cycle.

3. The method according to claim 1 or 2, wherein the elution captured in the first cycle is collected.

4. The method according to any of claims 1-3, wherein the chromatography system (49) is a single column chromatography system.

5. The method according to any of claims 1-4, wherein the presence of the target product (27) over time is registered as an elution curve, and the initiation of the elution phase is determined from the elution curve.

6. A column chromatography system (49) configured with at least one affinity chromatography separation unit, the chromatography system being configured for cyclic purification process performed on a sample comprising a target product (27) and is provided with a control unit configured to control each cycle of the purification process including:
- loading feed onto the separation unit,
- washing the separation unit, and
- eluting the target product from the separation unit by providing a flow of an elution fluid over the separation unit and controlling fraction collection by selectively directing a fraction of the outlet flow from the separation unit containing the eluted target product to a subsequent production step,
wherein the control unit is further configured for:
registering the presence of the target product (27) in the outlet flow from each column over time,
determining trigger points for target product collection in relation to presence of target product (27) in the outlet flow, the trigger points comprising a start time T1 and a stop time T2 for target product collection in relation to the start timepoint E of the elution phase for each cycle,
a) setting (41) a first time period ΔC₀ based on the trigger points (31, 32) within which a fraction of the elution in the first cycle is captured (42);
b) evaluating (43) timing of the captured elution to identify trigger points (33, 34, 35, 36, 37, 38) and the corresponding next time period ΔC₁, ΔC₂, AC₃;
c) applying (44) the timing to capture and collect a fraction of the elution during the elution phase in the following cycle;
d) repeating steps b) - c) during the purification process.

7. The chromatography system (49) according to claim 6, wherein the control unit is further configured to use elution captured in the first cycle for calibration purposes to determine the timing of the elution during the elution phase for the following cycle.

8. The chromatography system (49) according to claim 6 or 7, wherein the control unit is configured to collect elution captured in the first cycle.

9. The chromatography system (49) according to any of claims 6-8, wherein the chromatography system is a single column chromatography system.

10. The chromatography system (49) according to any of the claims 6-9, wherein the control unit is further configured to register the presence of the target product (27) over time as an elution curve, and to determine the initiation of the elution phase from the elution curve.

11. A computer program for controlling fraction collection in a chromatography system (49), comprising instructions which, when executed on at least one processor, cause the at least one processor to carry out the method according to any of claims 1-5, by means of a system according to any of claims 6-10.

12. A computer-readable storage medium carrying a computer program according to claim 11, for controlling fraction collection in a chromatography system (49).

## Patentansprüche

1. Verfahren zum Steuern von Fraktionssammlung eines Zielprodukts (27) in einem Säulenchromatographiesystem (49), das mindestens eine Affinitätschromatographie-Trenneinheit umfasst, wobei das Chromatographiesystem (49) für zyklische Reinigung konfiguriert ist, die an einer Probe durchgeführt wird, die das Zielprodukt (27) umfasst, und wobei jeder Zyklus des Reinigungsprozesses beinhaltet:
- Beschicken der Trenneinheit mit Zuführung (25),
- Waschen der Trenneinheit und
- Eluieren des Zielprodukts aus der Trenneinheit durch Bereitstellen eines Stroms einer Elutionsflüssigkeit über die Trenneinheit und Steuern einer Fraktionssammlung durch selektives Leiten einer Fraktion des Auslassstroms aus der Trenneinheit, der das eluierte Zielprodukt enthält, zu einem nachfolgenden Produktionsschritt,
wobei das Verfahren zum Steuern von Fraktionssammlung des Zielprodukts Folgendes umfasst:
Registrieren des Vorhandenseins des Zielprodukts (27) im Auslassstrom aus jeder Säule mit der Zeit,
Bestimmen von Auslösepunkten für die Zielproduktsammlung in Bezug auf das Vorhandensein eines Zielprodukts (27) im Auslassstrom, wobei die Auslösepunkte eine Startzeit T1 und eine Stoppzeit T2 für die Zielproduktsammlung in Bezug auf den Startzeitpunkt E der Elutionsphase für jeden Zyklus umfassen, und wobei das Verfahren zum Steuern von Fraktionssammlung eines Zielprodukts weiter umfasst:
a) Festlegen (41) einer ersten Zeitspanne ΔC₀ basierend auf den Auslösepunkten (31, 32), innerhalb derer eine Fraktion der Elution im ersten Zyklus erfasst wird (42);
b) Auswerten (43) des Zeitplans der erfassten Elution, um Auslösepunkte (33, 34, 35, 36, 37, 38) und die entsprechende nächste Zeitspanne ΔC₁, ΔC₂, ΔC₃ zu identifizieren;
c) Anwenden (44) des Zeitplans, um eine Fraktion der Elution während der Elutionsphase im folgenden Zyklus zu erfassen und zu sammeln;
d) Wiederholen der Schritte b) - c) während des Reinigungsprozesses.

2. Verfahren nach Anspruch 1, wobei die im ersten Zyklus erfasste Elution zu Kalibrierungszwecken verwendet wird, um den Zeitplan der Elution während der Elutionsphase für den folgenden Zyklus zu bestimmen.

3. Verfahren nach Anspruch 1 oder 2, wobei die im ersten Zyklus erfasste Elution gesammelt wird.

4. Verfahren nach einem der Ansprüche 1-3, wobei das Chromatographiesystem (49) ein Einsäulenchromatographiesystem ist.

5. Verfahren nach einem der Ansprüche 1-4, wobei das Vorhandensein des Zielprodukts (27) mit der Zeit als Elutionskurve registriert wird, und das Initiieren der Elutionsphase aus der Elutionskurve bestimmt wird.

6. Säulenchromatographiesystem (49), das mit mindestens einer Affinitätschromatographie-Trenneinheit konfiguriert ist, wobei das Chromatographiesystem für einen zyklischen Reinigungsprozess konfiguriert ist, der an einer Probe durchgeführt wird, die ein Zielprodukt (27) umfasst, und mit einer Steuereinheit bereitgestellt ist, die konfiguriert ist, um jeden Zyklus des Reinigungsprozesses zu steuern, Folgendes beinhaltend:
- Beschicken der Trenneinheit mit Zuführung,
- Waschen der Trenneinheit und
- Eluieren des Zielprodukts aus der Trenneinheit durch Bereitstellen eines Stroms einer Elutionsflüssigkeit über die Trenneinheit und Steuern einer Fraktionssammlung durch selektives Leiten einer Fraktion des Auslassstroms aus der Trenneinheit, der das eluierte Zielprodukt enthält, zu einem nachfolgenden Produktionsschritt,
wobei die Steuereinheit weiter konfiguriert ist zum:
Registrieren des Vorhandenseins des Zielprodukts (27) im Auslassstrom aus jeder Säule mit der Zeit,
Bestimmen von Auslösepunkten für die Zielproduktsammlung in Bezug auf das Vorhandensein eines Zielprodukts (27) im Auslassstrom, wobei die Auslösepunkte eine Startzeit T1 und eine Stoppzeit T2 für die Zielproduktsammlung in Bezug auf den Startzeitpunkt E der Elutionsphase für jeden Zyklus umfassen,
a) Festlegen (41) einer ersten Zeitspanne ΔC₀ basierend auf den Auslösepunkten (31, 32), innerhalb derer eine Fraktion der Elution im ersten Zyklus erfasst wird (42);
b) Auswerten (43) des Zeitplans der erfassten Elution, um Auslösepunkte (33, 34, 35, 36, 37, 38) und die entsprechende nächste Zeitspanne ΔC₁, ΔC₂, ΔC₃ zu identifizieren
c) Anwenden (44) des Zeitplans, um eine Fraktion der Elution während der Elutionsphase im folgenden Zyklus zu erfassen und zu sammeln;
d) Wiederholen der Schritte b) - c) während des Reinigungsprozesses.

7. Chromatographiesystem (49) nach Anspruch 6, wobei die Steuereinheit weiter konfiguriert ist, um die im ersten Zyklus erfasste Elution zu Kalibrierungszwecken zu verwenden, um den Zeitplan der Elution während der Elutionsphase für den folgenden Zyklus zu bestimmen.

8. Chromatographiesystem (49) nach Anspruch 6 oder 7, wobei die Steuereinheit konfiguriert ist, um die im ersten Zyklus erfasste Elution zu sammeln.

9. Chromatographiesystem (49) nach einem der Ansprüche 6-8, wobei das Chromatographiesystem ein Einsäulenchromatographiesystem ist.

10. Chromatographiesystem (49) nach einem der Ansprüche 6-9, wobei die Steuereinheit weiter konfiguriert ist, um das Vorhandensein des Zielprodukts (27) mit der Zeit als Elutionskurve zu registrieren, und das Initiieren der Elutionsphase aus der Elutionskurve zu bestimmen.

11. Computerprogramm zum Steuern von Fraktionssammlung in einem Chromatographiesystem (49), das Anweisungen umfasst, die, wenn sie auf mindestens einem Prozessor ausgeführt werden, den mindestens einen Prozessor veranlassen, das Verfahren nach einem der Ansprüche 1-5 anhand eines Systems nach einem der Ansprüche 6-10 durchzuführen.

12. Computerlesbares Speichermedium, das ein Computerprogramm nach Anspruch 11 zum Steuern von Fraktionssammlung in einem Chromatographiesystem (49) trägt.

## Revendications

1. Procédé de commande de collecte de fractions d'un produit cible (27) dans un système de chromatographie sur colonne (49) incluant au moins une unité de séparation par chromatographie d'affinité, le système de chromatographie (49) étant configuré pour une purification cyclique effectuée sur un échantillon comprenant le produit cible ( 27), chaque cycle du processus de purification incluant :
- le chargement d'une charge (25) sur l'unité de séparation,
- le lavage de l'unité de séparation, et
- l'élution du produit cible depuis l'unité de séparation en fournissant un flux d'un fluide d'élution sur l'unité de séparation et la commande de collecte de fraction en dirigeant sélectivement une fraction du flux de sortie depuis l'unité de séparation contenant le produit cible élué jusqu'à une étape de production ultérieure,
dans lequel le procédé de commande de collecte de fraction du produit cible comprend :
l'enregistrement de la présence du produit cible (27) dans le flux de sortie depuis chaque colonne au fil du temps,
la détermination de points de déclenchement pour la collecte de produit cible par rapport à la présence du produit cible (27) dans le flux de sortie, les points de déclenchement comprennent un temps de début T1 et un temps d'arrêt T2 pour la collecte de produit cible par rapport au point temporel de début E de la phase d'élution pour chaque cycle, et dans lequel le procédé de commande de collecte de fraction de produit cible comprend en outre :
a) la définition (41) d'une première période de temps ΔC₀ sur la base des points de déclenchement (31, 32) au cours de laquelle une fraction de l'élution dans le premier cycle est capturée (42) ;
b) l'évaluation (43) de la synchronisation de l'élution capturée pour identifier des points de déclenchement (33, 34, 35, 36, 37, 38) et la période de temps suivante ΔC₁, ΔC₂, ΔC₃ correspondante ;
c) l'application (44) de la synchronisation pour capturer et collecter une fraction de l'élution pendant la phase d'élution du cycle suivant ;
d) la répétition des étapes b) à c) pendant le processus de purification.

2. Procédé selon la revendication 1, dans lequel l'élution capturée dans le premier cycle est utilisée à des fins d'étalonnage pour déterminer la synchronisation de l'élution pendant la phase d'élution pour le cycle suivant.

3. Procédé selon la revendication 1 ou 2, dans lequel l'élution capturée dans le premier cycle est collectée.

4. Procédé selon l'une quelconque des revendications 1-3, dans lequel le système de chromatographie (49) est un système de chromatographie sur colonne unique.

5. Procédé selon l'une quelconque des revendications 1-4, dans lequel la présence du produit cible (27) au fil du temps est enregistrée en tant que courbe d'élution, et le début de la phase d'élution est déterminé à partir de la courbe d'élution.

6. Système de chromatographie sur colonne (49) configuré avec au moins une unité de séparation par chromatographie d'affinité, le système de chromatographie étant configuré pour un processus de purification cyclique effectué sur un échantillon comprenant un produit cible (27) et est pourvu d'une unité de commande configurée pour commander chaque cycle de le processus de purification incluant :
- le chargement d'une charge sur l'unité de séparation,
- le lavage de l'unité de séparation, et
- l'élution du produit cible depuis l'unité de séparation en fournissant un flux d'un fluide d'élution sur l'unité de séparation et la commande de collecte de fraction en dirigeant sélectivement une fraction du flux de sortie depuis l'unité de séparation contenant le produit cible élué jusqu'à une étape de production ultérieure,
dans lequel l'unité de commande est en outre configurée pour :
l'enregistrement de la présence du produit cible (27) dans le flux de sortie depuis chaque colonne au fil du temps,
la détermination des points de déclenchement pour la collecte de produit cible par rapport à la présence du produit cible (27) dans le flux de sortie, les points de déclenchement comprenant un temps de début T1 et un temps d'arrêt T2 pour la collecte de produit cible par rapport au point temporel de début E de la phase d'élution pour chaque cycle,
a) la définition (41) d'une première période de temps ΔC₀ sur la base des points de déclenchement (31, 32) au cours de laquelle une fraction de l'élution dans le premier cycle est capturée (42) ;
b) l'évaluation (43) de la synchronisation de l'élution capturée pour identifier des points de déclenchement (33, 34, 35, 36, 37, 38) et la période de temps suivante ΔC₁, ΔC₂, ΔC₃ correspondante
c) l'application (44) de la synchronisation pour capturer et collecter une fraction de l'élution pendant la phase d'élution du cycle suivant ;
d) la répétition des étapes b) à c) pendant le processus de purification.

7. Système de chromatographie (49) selon la revendication 6, dans lequel l'unité de commande est en outre configurée pour utiliser l'élution capturée dans le premier cycle à des fins d'étalonnage afin de déterminer la synchronisation de l'élution pendant la phase d'élution pour le cycle suivant.

8. Système de chromatographie (49) selon la revendication 6 ou 7, dans lequel l'unité de commande est configurée pour collecter l'élution capturée dans le premier cycle.

9. Système de chromatographie (49) selon l'une quelconque des revendications 6-8, dans lequel le système de chromatographie est un système de chromatographie sur colonne unique.

10. Système de chromatographie (49) selon l'une quelconque des revendications 6-9, dans lequel l'unité de commande est en outre configurée pour enregistrer la présence du produit cible (27) au fil du temps en tant que courbe d'élution, et pour déterminer le début de la phase d'élution à partir de la courbe d'élution.

11. Programme informatique pour commander la collecte de fraction dans un système de chromatographie (49), comprenant des instructions qui, lorsqu'elles sont exécutées sur au moins un processeur, amènent le au moins un processeur à réaliser le procédé selon l'une quelconque des revendications 1-5, à l'aide d'un système selon l'une quelconque des revendications 6-10.

12. Support de stockage lisible par ordinateur portant un programme informatique selon la revendication 11, pour commander la collecte de fraction dans un système de chromatographie (49).
